# EUROPEAN PATENT APPLICATION

(11) **EP 2 471 912 A1**
(43) Date of publication of application: **04.07.2012**
(21) Application number: 10811717.7
(22) Date of filing: 17.08.2010
(51) Int. Cl.: C12N 9/24, C12N 1/14, C12P 19/14, C12R 1/885

(54) **B-GLUCANASE AND XYLANASE PREPARATION METHOD USING WASTE FUNGI, AND LIQUID CULTURE MEDIUM**

(30) Priority: 24.08.2009 JP 2009192993
(71) Applicant: Asahi Group Holdings, Ltd., Tokyo 130-8602 (JP)
(72) Inventor: FUKUDA, Kazuro, Moriya-shi Ibaraki 302-0106 (JP)
(74) Representative: Müller, Christian Stefan Gerd
(86) International application number: PCT/JP2010/063838
(87) International publication number: WO 2011/024667

(57) **Abstract**

Disclosed is production of cellulase having excellent ability to decompose cellulosic resources containing xylan at low cost.

A method for producing β-glucanase and xylanase, comprising the step of culturing a microorganism classified under the genus Trichoderma by using a liquid culture medium which contains fungus body debris as an organic carbon source.

## Description

### TECHNICAL FIELD

The present invention relates to a method for simultaneously and highly producing β-glucanase and xylanase, and a liquid culture medium useful for producing the enzymes.

### BACKGROUND ART

In order to effectively utilize cellulosic resources, a method for efficiently decomposing cellulose has been explored in recent years. Cellulose is mainly decomposed by microorganisms in nature, and it is known that various microorganisms such as bacteria, filamentous fungi and the like produce cellulolytic enzymes.

These microorganisms secrete the cellulolytic enzymes outside those fungus bodies, and cellulose is decomposed by its action into glucose via mainly cello-oligosaccharide and cellobiose. Cellulolytic enzymes are generally called cellulase.

When cellulase is intended to be artificially produced, the genus Trichoderma is known as microorganisms secreting cellulase, and is widely utilized. Moreover, a method for secreting cellulase by culturing the microorganisms classified under the genus Trichoderma by using a culture medium containing nutrients such as a carbon source, a nitrogen source and the like is also known.

However, in the conventional method for producing cellulase, materials usable as a carbon source are limited.
For example, crystalline cellulose is expensive, and even if inexpensive cellulosic resources are used, they generally require pretreatment such as heat treatment, alkali treatment and the like, that causes relatively high cost.

Patent Literature 1, for example, discloses a substrate for producing cellulase, which substrate is obtained by boiling used paper in a ferrous sulfate solution and can be inoculated with cellulase-producing microorganisms. In addition, Patent Literature 2 discloses a method for producing a substrate for producing cellulase, which substrate can be inoculated with Trichoderma reesei which is a cellulase-producing microorganism, by boiling pulverized bagasse with caustic alkali and treating with a hypochlorite solution.

In addition, the cellulase obtained by these conventional methods mainly contains β-glucanase, and has low xylanase activity and little ability to decompose cellulosic resources containing xylan, such as bagasse, rice straw and the like. Therefore, it is less effective for the purpose of effectively utilizing a variety of naturally-occurring cellulosic resources.

Patent Literature 3 discloses a method for producing cellulase, which method includes the step of collecting cellulase obtained by liquid culture of mutants of Trichoderma reesei. As carbon sources of media, a variety of materials with different chemical structures and characters such as cellulose powder, cellobiose, filter paper, general paper, sawdust, bran, chaff, bagasse, soymeal, coffee grounds and the like are listed (paragraph 0011).

Among these, however, only cellobiose (Example 1) and Avicel (Example 2) are actually used for culturing operations, and production of cellulase has not been confirmed in the other materials, i.e. natural cellulosic materials.

Patent Literature 4 discloses a method for producing xylanase by culturing a microorganism classified under the genus Trichoderma by using a diluted alcohol distillation waste fluid of rye subjected to preliminary treatment such as removal of solid constituents, concentration of nonvolatile components, autoclave treatment of the concentrate and the like.

However, rye used as a carbon source in this technique is not easily available, and more complicated pretreatment is required. Thus, the technique causes high cost. In addition, the production amount of β-glucanase is rather decreased by this method.

On the other hand, when they are manufactured medical ingredients or nutrient supplemental ingredients, health recourse ingredients, tasty ingredients, raw materials and intermediates for foods and drugs and the like with culturing microorganisms, they have been disposed the fungus body, the culture medium and the like left to remain after the collection of intended substances. However, considerable labors and costs are needed for the disposal of such fungus body and culture medium, because the fungus body and the culture medium must be detoxified by way of inactivation or separation treatments so as not to burden adverse influences on the environment.

To solve the problem, for example, Patent Literature 5 discloses that the fungus body debris left to remain after fermentation production with the use of the genus Propionibactor, the genus Pseudomonas and the like is recycled as a culture medium or a culture substrate for cultivating mushroom fruit-bodies.

However, it has not been known to utilize fungus body debris for a medium to simultaneously and highly produce β-glucanase and xylanase.

### PRIOR ART LITERATURE

### PATENT LITERATURE

[Patent Literature 1] Japanese Patent Laid-open Publication No. 2003-137901
[Patent Literature 2] Japanese Examined Patent Application Publication No. H5(1993)-33984
[Patent Literature 3] Japanese Patent Laid-open Publication No. H9(1997)-163980
[Patent Literature 4] Japanese Patent Laid-open Publication No. H11 (1999)-113568
[Patent Literature 5] Japanese Patent Laid-open Publication No. 2003-47338

### SUMMARY OF THE INVENTION

### PROBLEMS TO BE SOLVED BY THE INVENTION

The present invention solves the above conventional problems, and an object thereof is to produce cellulase which has excellent ability to decompose cellulosic resources containing xylan at low cost.

### MEANS FOR SOLVING THE PROBLEMS

As a result of intensive studies for a method for simultaneously and highly producing β-glucanase and xylanase, which decompose (glycosylate) cellulosic materials, and production of the enzymes, the present inventors found a method for simultaneously and highly producing β-glucanase and xylanase by culturing a microorganism classified under the genus Trichoderma by using fungus body debris as a nutrient for a liquid culture medium, and the liquid culture medium useful for producing these enzymes.

The present invention provides a method for producing β-glucanase and xylanase, comprising the step of culturing a microorganism classified under the genus Trichoderma by using a liquid culture medium which contains fungus body debris as an organic nitrogen source.

In one embodiment, the concentration of the fungus body debris in the liquid culture medium is not less than 1% W/V.

In one embodiment, the concentration of the fungus body debris in the liquid culture medium is from 2 to 10% W/V.

In one embodiment, a raw material for the fungus body debris is a microorganism classified under the genus Trichoderma.

In one embodiment, the microorganism classified under the genus Trichoderma is Trichoderma reesei.

In one embodiment, the liquid culture medium further contains a natural cellulose material as a carbon source, and ammonia nitrogen or amino nitrogen as a nitrogen source.

In one embodiment, the concentration of the natural cellulose material in the liquid culture medium is not less than 2% W/V.

In one embodiment, the natural cellulose material is at least one kind selected from the group consisting of pulp, residue of beer, residue of Mugi tea extract, wheat bran and marc of apple.

In one embodiment, the fungus body debris is added to the liquid culture medium in the course of culture.

In addition, the present invention provides a liquid culture medium comprising fungus body debris as an organic nitrogen source, for use in culture of a microorganism classified under the genus Trichoderma.

In one embodiment, the fungus body debris is contained at a concentration of not less than 1% W/V.

In addition, the present invention provides β-glucanase and xylanase produced by any of the above described methods.

In addition, the present invention provides a method for decomposing or glycosylating a cellulosic resource, characterized by using the β-glucanase and xylanase.

### EFFECTS OF THE INVENTION

The present invention contributes to the solution of the environmental issues, because the industrial wastes can be decreased in amount by making good use of fungus body debris. In addition, since β-glucanase and xylanase, i.e., cellulolytic enzymes, are simultaneously and highly produced, the present invention is extremely useful for glycosylation of natural cellulosic resources such as bagasse, rice straw and the like. Specifically, it is useful for biomass ethanol production in which ethanol is produced from cellulosic resources.

### BRIEF DESCRIPTION OF DRAWINGS

[Fig. 1] A graph showing changes in enzyme activity of culture supernatant fluids against concentrations of fungus body debris in liquid culture media containing 3% of copy paper.
[Fig. 2] A graph showing changes in enzyme activity of culture supernatant fluids against concentrations of fungus body debris in liquid culture media containing 1% of crystalline cellulose.
[Fig. 3] A graph showing comparison of concentrations of produced glucose, when rice straw is glycosylated, using the respective supernatant fluids of the liquid culture medium containing 1.5% of the fungus body debris obtained in Example 1 and the liquid culture medium containing 1.5% of the fungus body debris obtained in Reference Example 1.
[Fig. 4] A graph showing comparison of concentrations of produced glucose, when a cellulose raw material is glycosylated, using the respective supernatant fluids of the culture medium containing 1.5% of the fungus body debris obtained in Example 1 and the culture medium containing 1.5% of the fungus body debris obtained in Reference Example 1.
[Fig. 5] A graph showing enzyme activity obtainable from 0.2% of polypeptone, and changes in enzyme activity of culture supernatant fluids against concentrations of fungus body debris, in liquid culture media containing 3% of residue of beer.
[Fig. 6] A graph showing enzyme activity obtainable from 0.2% of polypeptone, and changes in enzyme activity of culture supernatant fluids against concentrations of fungus body debris, in liquid culture media containing 5% of residue of Mugi tea extract.
[Fig. 7] A graph showing changes in enzyme activity of culture supernatant fluids against concentrations of fungus body debris in liquid culture media containing 5% of wheat bran.
[Fig. 8] A graph showing changes in enzyme activity of culture supernatant fluids against concentrations of fungus body debris in liquid culture media containing 4% of apple marc.
[Fig. 9] A graph showing changes in enzyme activity of culture supernatant fluids against concentrations of corn steep liquor in liquid culture media containing 3% of copy paper.
[Fig. 10] A graph showing changes in enzyme activity of culture supernatant fluids against concentrations of polypeptone in liquid culture media containing 3% of copy paper.

### EMBODIMENT FOR CARRYING OUT THE INVENTION

### Liquid Culture Medium

The liquid culture medium of the present invention is a material which contains nutrients for growing a microorganism classified under the genus Trichoderma. Such a liquid culture medium is prepared based on a liquid culture medium (generally called a Mandel medium) obtained by dissolving and suspending nutrients for the medium in 100 ml of water. The liquid culture medium contains water as a medium, fungus body debris and the like as an organic nitrogen source, optionally a natural cellulose material and the like as a carbon source, and optionally ammonia nitrogen or amino nitrogen and the like as a nitrogen source. One example of the preferred medium compositions of the present invention is shown below.

Medium composition (the present invention):
Fungus body debris: 1 g, a natural cellulose material: 3 to 5 g, (NH₄)₂SO₄: 0.14 g, KH₂PO₄: 1.5 g, CaCl₂•2H₂O: 0.03g, MgSO₄•7H₂O: 0.03 g, Tween 80: 0.1 mL, a trace element liquid (liquid of H₃BO₄ 6 mg, (NH₄)₆Mo₇O₂₄•4H_{2O} 26 mg, FeCl₃•6H₂O 100 mg, CuSO₄•5H₂O 40 mg, MnCl₂•4H₂O 8 mg and ZnSO₄•7H₂O 200 mg): 0.1 mL, and water: 100 mL are contained (adjusted to pH 4.8 using phosphoric acid or sodium hydroxide).

As a reference, one of typical examples of a medium composition of a Mandel medium is described below.

Medium composition (Mandel medium):
Polypeptone: 0.2 g, crystalline cellulose (trade name Avicel PH101 manufactured by Fluka BioChemika): 1 g, (NH₄)₂SO₄: 0.14 g, KH₂PO₄: 1.5 g, CaCl₂•2H₂O: 0.03g, MgSO₄•7H₂O: 0.03 g, Tween 80: 0.1 mL, a trace element liquid (liquid of H₃ B0₄ 6 mg, (NH₄)₆Mo₇O₂₄•4H₂O 26 mg, FeCl₃•6H₂O 100 mg, CuSO₄•5H₂O 40 mg, MnCl₂•4H₂O 8 mg and ZnSO₄•7H₂O 200 mg): 0.1 mL, and water: 100 mL are contained (adjusted to pH 4.8 using phosphoric acid or sodium hydroxide).
In this connection, corn steep liquor may be used instead of polypeptone as the organic nitrogen source.

The fungus body debris means a residue left to remain after collection of an intended substance in production of a substance such as an enzyme which is produced with growing a microorganism, or means a processed material of the residue. The microorganism as a raw material for the fungus body debris is not particularly limited, if it is in the class confirmed to be safe to human bodies.

Examples thereof are microorganisms classified under genus Trichoderma, genus Aspergillus, genus Acremonium, genus Sporotrichum, genus Penicillium, genus Talaromyces, genus Humicola, genus Neocallimastix, genus Thermomyces or genus Clostridium, and genus Streptomyces, which are safe to human bodies and are usable for the liquid culture medium of the present invention. Preferable among those are microorganisms classified under genus Trichoderma. A preferable microorganism classified under genus Trichoderma is Trichoderma reesei or Trichoderma viride, of which Trichoderma reesei is particularly preferable.

To produce the fungus body debris, firstly, a microorganism as a raw material is planted on a suitable medium and is then cultured under suitable conditions to obtain a culture solution. The medium for use in culture is preferably a liquid culture medium such as Mandel medium. This is because it is easy to separate the fungus body from the culture liquid. In a preferred embodiment, the culture for producing the fungus body debris is carried out in the same manner as employed in the method of the present invention which will be described below in the description of the present invention.

Then, the culture liquid resulting from the culture is separated into the supernatant fluid and the fungus body. The separation method may be a known one, for example, a filtration method, a centrifugal separation method and the like can be exemplified. The culture liquid and the supernatant fluid are heat sterilized, for example, at 121°C for 15 minutes, in an autoclave, before or after the step of separating to kill the microorganism. The heat sterilization of the microorganism may be done before addition to a medium; or a medium may be subjected to heat sterilization after the addition of the microorganism. The heating step or the separating step may be carried out several times.

The wet fungus body residue separated from the culture liquid may be directly used as the fungus body debris. The wet fungus body may be dried to obtain dried fungus body residue, which may be used as fungus body debris. As the drying method, there may be employed a conventional method, for example, a natural drying method, a hot air drying method, a vacuum drying method, spray drying method and the like.

The concentration of the fungus body debris in the liquid culture medium is preferably not less than 1% W/V. The concentration of the fungus body debris in the liquid culture medium is more preferably from 2 to 10% W/V, still more preferably from 2 to 8% W/V, particularly from 3 to 6% W/V. When the concentration of the fungus body debris is less than 1% W/V, the production amount of cellulase, especially xylanase, may not increase so much.

The natural cellulose material means a water-insoluble cellulose which retains a naturally-existing molecular structure as it is. Examples of the natural cellulose material include paper, pulp, residue of beer, residue of Mugi tea extract, wheat bran, marc of fruits such as apple and the like. On the other hand, a crystalline cellulose such as Cellobiose or Avicel is a pure chemical compound obtained by decomposing a cellulose with a cellulase, and purifying it to thereby cause it to have a specific structure. The crystalline cellulose is not included in the natural cellulose materials herein referred to.

Pulp means fibers for use as a raw material for paper. The pulp is preferably in the class of chemical pulp or waste paper pulp, those having a high cellulose purity. The pulp is preferably pulp derived from paper, obtained by decomposing or cutting the paper.

Preferable examples of the paper are high-quality paper, low-quality paper, copy paper, newspaper and corrugated paper. The paper may be any one, if it contains preferable pulp, and it may be printed paper, written-down paper or so-called waste paper. For example, there may be used old books, magazines, sheets of used notebooks, leaflets, envelopes, letter paper, post cards, tissues and the like.

The concentration of the pulp in the liquid culture medium is preferably not less than 2% W/V. When the concentration of the pulp is less than 2% W/V, the production amount of cellulase, especially β-glucanase, may not increase so much. The concentration of the pulp in the liquid culture medium is more preferably not less than 3% W/V, still more preferably not less than 4% W/V, not less than 5% W/V, not less than 6% W/V or not less than 7% W/V. Preferably, the paper should be cut with a shredder to facilitate stirring and mixing of the liquid culture medium.

The residue of beer is a residue obtained from that barley is germinated to obtain malt, it is glycosylated, and the resulting sweet wort is removed by filtration, which is by-produced in the course of manufacturing of beer. There is no limitation in selection of the kind of barley, subsidery raw materials and the like. A residue of a by-product obtained in the course of manufacturing of low-malt beer which contains a decreased ratio of malt is also included in the examples of the residue of beer of the present invention.

A large amount of the residue of beer is produced in the course of manufacturing of beer, and thus, the residue of beer is easily available. The residue of beer is a by-product of food manufacturing, and thus is safe and excellent in sanitary quality, since the raw materials for such foods are strictly inspected as for their quality, and since the production steps therefor are strictly controlled. Examples of the residue of beer include crude residue of beer, dewatered residue of beer, and dried residue of beer.

The initial concentration of the residue of beer in the liquid culture medium is preferably not less than 2% W/V. When the concentration of the residue of beer is less than 2% W/V, the production amount of cellulase, especially β-glucanase, may not increase so much. The concentration of the residue of beer in the liquid culture medium is more preferably not less than 3% W/V, still more preferably not less than 4% W/V, not less than 5% W/V, not less than 6% W/V or not less than 7% W/V.

The residue of Mugi tea extract means a residue left to remain after extraction of a water-soluble ingredient from roasted Mugi grains with water or other solvent. The residue of Mugi tea extract is produced in a large amount in the course of manufacturing of Mugi tea and thus is easily available. The residue of Mugi tea extract is a by-product food manufacturing, and thus is excellent in sanitary quality and safe, since raw materials for such foods are strictly inspected as for their quality, and since the production steps therefor are strictly controlled.

The kind of Mugi as a raw material for the residue of Mugi tea extract is not limited, if the use thereof is suitable for production of Mugi tea. Barley is generally used to produce Mugi tea. Examples thereof include six-rowed barley, two-rowed barley, naked barley oats and the like, among which six-rowed barley and two-rowed barley are preferable. These may be mixed for use.

To manufacture the residue of Mugi tea extract, firstly, grains of Mugi such as barley and the like are roasted. Examples of the roasted method generally include hot-air roasting, hot sand roasting or far infrared roasting and the like. The roasting temperature is from 100 to 700°C, preferably from 200 to 600°C, and the roasting time is from 1 to 60 minutes, preferably from 5 to 60 minutes.

After that, the roasted Mugi grains are immersed in an extraction solvent, and are then heated to, preferably, 80°C or higher. Water is generally used as the solvent. A water-soluble ingredient in the Mugi grains is extracted into water by boiling the Mugi grains in hot water. The water-soluble ingredient extracted from the Mugi grains contains a flavor component, a starch component and the like.

The extraction time is not limited, and is preferably from 20 minutes to one hour.

Then, the extracted liquid is separated as Mugi tea, and the rest is a residue of Mugi tea extract. Separation of the extracted liquid may be made by a method conventionally employed such as decantation, filtration, centrifugal separation and the like. The residue of Mugi tea extract may optionally be subjected to a treatment such as washing, dewatering, drying and the like.

The concentration of the residue of Mugi tea extract in the liquid culture medium is preferably not less than 3% W/V. When the concentration of the residue of Mugi tea extract is less than 3% W/V, the production amount of cellulase, especially β-glucanase, may not increase so much. The concentration of the residue of Mugi tea extract in the liquid culture medium is more preferably not less than 4% W/V, still more preferably not less than 5% W/V, not less than 6% W/V, not less than 7% W/V or not less than 8% W/V.

The wheat bran means a mixture of hulls and germs of wheat grains. On the other hand, wheat flour is obtained by removing the bran (i.e., hulls and germs) from wheat grains, and grinding the resulting wheat grains. The wheat bran is produced in a large amount as a by-product in the course of industrial manufacturing of edible wheat flour and thus is easily available. The wheat bran is a by-product of a food and thus is excellent in sanitary quality and safe, since the quality inspection of the raw materials therefor and control of the production steps are strictly made. Therefore, the wheat bran is preferably used in the method of the present invention.

The kind of wheat for use in preparation of the wheat bran is not particularly limited. Examples thereof include Hokushin, Fukusayaka, Norin 61, Nanbukomugi, Kitanokaori, Haruyutaka, Haru yo Koi and the like.

The wheat bran is generally in the form of flakes. Flake-like wheat bran may be used as it is. The wheat bran may be appropriately ground to obtain fine particles thereof for use; or the wheat bran may be granulated to form a mass of particles for use. The form of the wheat bran may be, for example, large bran, small bran, flour or the like. As the wheat bran, there may be used food materials, health food and the like as commercially available products.

The concentration of the wheat bran in the liquid culture medium is preferably not less than 3% W/V. When the concentration of the wheat bran is less than 3% W/V, the production amount of cellulase, especially β-glucanase, may not increase so much. The concentration of the wheat bran in the liquid culture medium is more preferably not less than 4% W/V, still more preferably not less than 5% W/V, not less than 6% W/V, not less than 7% W/V or not less than 8% W/V.

The marc of fruits is a residue obtained from that fruits are squeezed, and the resulting fruit juice is removed by filtration, which is by-produced in the course of manufacturing of juice and the like. The marc of fruits can be obtained in a large amount in the course of manufacturing of juice and the like, and thus is easily available. The marc of fruits is a by-product of food manufacturing and thus is excellent in sanitary quality and safe, since the quality inspection of the raw materials therefore and control of the production steps are strictly done. The marc of fruits is preferably a marc of rosaceous fruits such as apple, pear, peach, cherry, strawberry and the like. Particularly, marc of apple is preferably used, because the use thereof enables production of large amounts of the desired enzymes.

There is no limitation in selection of the kind of the apple, insofar as it has been conventionally used to manufacture apple juice. Examples of the apple include "Fuji", "Tsugaru", "Ohrin", "Jonagold", "Star King Delicious", "Mutsu" and the like. The apple fruit to be used may be fully-ripened or under-ripened.

To produce the marc of fruits, firstly, fruits are washed. In this step, some fruits unsuitable for use as a raw material are removed. The washed fruits are supplied into a crusher and are then crushed. The crushed fruits are supplied into a hydraulic juice-extracting machine by the use of a pump or the like to extract the juice thereof. Then, the marc of fruits is collected from the juice-extracting machine. The marc of fruits optionally may be washed, dewatered and dried.

The concentration of the marc of fruits in the liquid culture medium is preferably not less than 2% W/V. When the concentration of the marc of fruits is less than 2% W/V, the production amount of cellulase, especially β-glucanase, may not increase so much. The concentration of the marc of fruits in the liquid culture medium is more preferably not less than 3% W/V, still more preferably not less than 4% W/V, not less than 5% W/V or not less than 6% W/V.

The higher the concentration of the natural cellulose material in the liquid culture medium, the better. In other words, the upper limit of an amount of the natural cellulose material is such that the liquid culture medium can be stirred and mixed. If the liquid culture medium can not be stirred, microorganisms can not be uniformly mixed in the liquid culture medium, with the result that the culture thereof can not normally proceed. The upper limit of the concentration of pulp in the liquid culture medium may be 20, 15, 10 or 8% W/V, depending on the performance of the stirrer.

The natural cellulose material other than paper and pulp, for example, the residue of beer, the residue of Mugi tea extract, the wheat bran or the marc of fruits is preferably pre-treated when introduced into the liquid culture medium. Preferred pretreatment is, for example, pulverization treatment and delignification treatment. When lignin has been removed from the natural cellulose material, the strong cell walls are destroyed, so that cellulose can be easily utilized. Thus, enzymes are easily produced. Also, the delignification treatment can be more efficiently carried out by the pulverization treatment of the natural cellulose material.

The method for the delignification treatment is not particularly limited, and examples thereof include a method of decomposing the natural cellulose material by heating it at a high temperature in the presence of a strongly alkaline substance such as sodium hydroxide or in the presence of a strongly acidic substance such as sulfuric acid or phosphoric acid; a method of decomposing the natural cellulose material by the use of microorganisms; and a method of decomposing the natural cellulose material by a hydrothermal treatment under high temperature and high pressure. The method of decomposing the natural cellulose material by the hydrothermal treatment under high temperature and high pressure is preferred, from the viewpoint of a burden on the treatment equipment and the environment.

A conventional pretreatment usually made on raw materials for liquid culture media such as heat sterilization or the like further may be done.

The ammonia nitrogen refers to nitrogen contained in ammonia or ammonium salts derived from ammonia. The amino nitrogen refers to nitrogen contained in amines or amino compounds derived from amines. Examples of compounds which contain ammonia nitrogen or amino nitrogen include ammonium sulfate, ammonium nitrate, diammonium phosphate, ammonium chloride, aqueous ammonia, urea, and amino acids and salts thereof (e.g. leucine and sodium glutamate).

Among those, a particularly preferred compound to be used as the nitrogen source for use in the liquid culture medium of the present invention is ammonium sulfate. The reason is that ammonium sulfate is inexpensive and is easily available.

The concentration of the ammonia nitrogen or amino nitrogen in the liquid culture medium is from 30 to 660 mM as the number of moles of ammonium. Preferably, the concentration is from 40 to 580 mM. When the concentration is less than 30 mM, the production amount of cellulase, especially β-glucanase, may not increase so much. When the concentration exceeds 660 mM, productivity of the enzymes tends to decrease. It is preferred that the concentration of the ammonia nitrogen or amino nitrogen in the liquid culture medium is increased or decreased in accordance with the concentration of the natural cellulose material in the liquid culture medium. For example, when the concentration of the natural cellulose material is 4% W/V, the concentration of the ammonia or amino nitrogen is preferably 50 mM in view of cost and the like.

### Method for Producing β-Glucanase and Xylanase

Filamentous fungi classified under the genus Trichoderma are known as the fungi which can produce cellulase necessary for glycosylation of cellulose. The microorganisms classified under the genus Trichoderma for use in the present invention are not particularly limited as long as they can produce cellulase. A preferred microorganism classified under the genus Trichoderma is Trichoderma reesei or Trichoderma viride. Trichoderma reesei is particularly preferred.

Mycological properties of the filamentous fungi, i.e., Trichoderma reesei and Trichoderma viride are described in, for example, E.G. Simmons, Abst. 2nd International Mycological Congress, Tampa, Florida, U.S., August 1977, page 618.

A conventional aeration-agitation culture device is used in the liquid culture, which is performed with using the liquid medium of the present invention at a culture temperature of 20 to 33°C, preferably 28 to 30°C, at a culture pH of 4 to 6 for 4 to 10 days. When the above liquid culture medium is used from the beginning of the culture, concentrations of the ingredients (e.g. a carbon source and a nitrogen source) contained in the liquid culture medium correspond to the initial concentrations of the above ingredients in the culturing method of the present invention.

The fungus body debris may be added to the liquid culture medium in the course of the culture. This is because the production efficiency of cellulase may be improved by supplementing the fungus body debris, since the fungus body debris in the culture medium is decomposed with the proceeding of the culture.

When the fungus body debris is added, the natural cellulose material, ammonia nitrogen or amino nitrogen may be appropriately added in the course of the culture as required.

Subsequently, the fungus body is removed from the culture liquid by a known method such as centrifugation, filtration or the like to obtain a culture supernatant fluid of the filamentous fungus of the genus Trichoderma. The culture liquid or culture supernatant fluid of the filamentous fungus of the genus Trichoderma contains the intended cellulase, i.e. β-glucanase and xylanase, in high concentrations.

The β-Glucanase activity of the obtained culture liquid or culture supernatant fluid is not less than 30 U/mL, preferably not less than 50 U/mL, more preferably not less than 60 U/mL, still more preferably not less than 70 U/mL. Also, the xylanase activity of the culture liquid or culture supernatant fluid is not less than 25 U/mL, preferably not less than 30 U/mL, more preferably not less than 40 U/mL, still more preferably not less than 50 U/mL. When either the β-glucanase activity or the xylanase activity of the culture liquid or culture supernatant fluid falls below the above lower limit, effects on the purpose of effectively utilizing a variety of naturally-occurring cellulosic resources decrease.

The above hemicellulase activity can be quantified based on an increase in absorbance at 540 nm by reacting the reducing sugars produced by enzymatic hydrolysis of xylan derived from "oat spelts" as a substrate with DNS.

More specifically, to 1.9 mL of a 1% xylan substrate solution ("Xylan, from oat spelts" manufactured by Sigma is dissolved in a 200 mM acetic acid buffer solution (pH 4.5)), 0.1 mL of the culture liquid or culture supernatant fluid is added, and the obtained solution is enzymatically reacted at 40°C for exactly 10 minutes. Then, 4 mL of a DNS reagent (containing 0.75% dinitrosalicylic acid, 1.2% sodium hydroxide, 22.5% potassium sodium tartrate tetrahydrate, and 0.3% lactose monohydrate) is added thereto, and the obtained solution is mixed well to stop the reaction. In order to quantify the amount of reducing sugars contained in the reaction stop solution, the reaction stop solution is heated in a boiling-water bath for exactly 15 minutes. Next, the reaction stop solution is cooled to room temperature, and the amount of reducing sugars corresponding to xylose is then quantified by determining absorbance at 540 nm. One unit of the hemicellulase activity is represented as the enzyme amount which produces the reducing sugars corresponding to 1 µmol of xylose in 1 minute under the reaction conditions of 40°C and 10 minutes.

The passage of "culturing a microorganism classified under the genus Trichoderma" referred to in the present invention means an operation of growing the microorganism according to common techniques. That is, in a method of performing liquid culture for the purpose of producing β-glucanase and xylanase, if there is at least a process in which a microorganism classified under the genus Trichoderma grows in the above liquid culture medium of the present invention, the culturing method corresponds to the method of the present invention.

When the culture is performed, nutrients in the liquid culture medium decrease since a microorganism classified under the genus Trichoderma consumes them.
Thus, the concentrations of a carbon source and a nitrogen source (including an organic nitrogen source) in the culture medium at the end of the culture are under the given concentrations. Consequently, a microorganism classified under the genus Trichoderma may grow in a culture medium which does not correspond to the liquid culture medium of the present invention. Even under the above situation, when a liquid culture medium to be used corresponds to the liquid culture medium of the present invention containing a carbon source and a nitrogen source at given concentrations at, for example, the initiation of culture, the microorganism classified under the genus Trichoderma grow in the liquid culture medium of the present invention at least at the beginning of culture. Therefore, the culturing method clearly corresponds to the method of the present invention.

When a carbon source is contained in a large amount, particularly, at the beginning of culture, as described above, it is preferred that the upper limits of the concentrations of the carbon source and the nitrogen source should be limited to a certain level, from the viewpoint of convenience for stirring and mixing the liquid culture medium.

On the contrary, even if the concentration of the carbon source or the nitrogen source in the culture medium at the beginning of the culture is lower than a predetermined concentration, and if the culture is performed using a culture medium which does not correspond to the liquid culture medium of the present invention, this culturing method corresponds to the method of the present invention. The reason therefor is that, for example, when the concentration of the carbon source or the nitrogen source in the culture medium exceeds the predeterrmined concentration by supplementing it later, a microorganism classified under the genus Trichoderma grows in this liquid culture medium.

### Method for Decomposing or Glycosylating Cellulose Raw Materials

The β-glucanase and xylanase obtained by the method of the present invention are useful for decomposing or glycosylating cellulose raw materials. The cellulose raw materials referred to herein may be either synthetic cellulosic or natural cellulosic resources. The synthetic cellulose represents cellulose distributed as cellulose powder. The natural cellulosic resources include bagasse, rice straw, wheat straw, beer draff, wood and the like. The present invention can simultaneously and highly produce β-glucanase and xylanase, thus it excels in glycosylating natural cellulosic resources, specifically, bagasse, rice straw, wheat straw, beer draff and the like.

The method for decomposing or glycosylating cellulose raw materials can be performed using known methods, and is not particularly limited. One example includes a method in which cellulose raw materials are suspended in an aqueous medium as a substrate, and then the above culture liquid or culture supernatant fluid is added thereto, followed by performing glycosylation reaction by heating while stirring or shaking. In place of the above described culture liquid or culture supernatant fluid which shows cellulolytic activity, dry matters thereof or solutions obtained by dispersing or dissolving the dry matters in water may also be used.

It is preferred that the cellulose raw materials be preliminarily delignified. The reaction conditions such as a suspending method, a stirring method, a method for adding the above mixed solution, the order of addition, concentrations thereof and the like are appropriately adjusted to obtain glucose in higher yield.

The pH and temperature of the reaction solution may be within the range in which enzymes are not deactivated, and generally, when the reaction is carried out under normal pressure, the temperature and pH may be in the range of 30 to 70°C and of 3 to 7, respectively. In addition, the pressure, temperature and pH are appropriately adjusted to obtain glucose in higher yield as described above, and it is preferred that the reaction be carried out in an acetic acid- or phosphate-buffer solution under normal pressure at a temperature of 50 to 60°C and a pH of 4 to 6.
The reaction time is generally from 6 to 147 hours, and preferably from 24 to 72 hours.

An aqueous solution containing glucose is obtained by glycosylation of cellulose. The obtained aqueous solution can be subjected to purification treatment such as decolorization, desalination, enzyme removal and the like as necessary. The purification method is not particularly limited as long as it is a known method. For example, activated carbon treatment, ion-exchange resin treatment, chromatography treatment, filtration treatments such as microfiltration, ultrafiltration, reverse osmosis filtration and the like, crystallization treatment and the like may be used. These may be used alone or two or more may be used in combination.

The aqueous solution mainly composed of glucose purified by the above method can be used as it is, and may be solidified by drying as necessary. The drying method is not particularly limited as long as it is a known method. For example, spray drying, freeze drying, drum drying, thin-film drying, tray drying, flash drying, vacuum drying and the like may be used. These may be used alone or two or more may be used in combination.

### EXAMPLES

The present invention will now be described in more detail by way of Examples, but the present invention is not limited thereto.

### Example 1

Trichoderma reesei QM9414 was planted on a Mandel culture medium and was cultured under the same conditions as those described in the present Example to obtain a culture liquid. The obtained culture liquid was treated with a centrifugal separation apparatus ("Avanti HP-25" manufactured by BECMAN COULTER) to collect the fungi. The residual fungus body was dried at about 60°C for about 24 hours to obtain fungus body debris.

Trichoderma reesei QM9414 (NBRC 31329) was cultured on a potato dextrose agar medium at 28°C for 7 days to sufficiently form spores. In a Mandel medium, crystalline cellulose as a carbon source was replaced with 3% of copy paper (3 g/100 mL); 1% of ammonium sulfate as an inorganic nitrogen source was added thereto; and polypeptone as an organic nitrogen source was replaced with the above-obtained fungus body debris, and the fungus body debris were added so that their concentrations could be 0.5%, 1.0%, 1.5%, 2.0% and 3.0%, respectively. The obtained solutions were adjusted to pH 4.8 using phosphoric acid or sodium hydroxide to obtain liquid culture media; and 100 mM of each of the liquid culture media was charged in a 500 mL baffled Erlenmeyer flask and was then sterilized in an autoclave by heating at 121°C for 15 minutes. Then, a loopful of the cultured Trichoderma reesei was inoculated into the liquid culture medium, and the fungi were cultured at 28°C and at 180 rpm for 7 days while being shaken. The culture liquid was centrifuged on the seventh day, and the β-glucanase activity and xylanase activity of the supernatant fluid were determined.

### (Determination of Enzyme Activity)

Enzyme activity of each of the culture liquids obtained in the above step was determined.

For β-glucanase activity, absorbance of a dyed fragment generated by enzymatic decomposition of dye-labeled β-glucan as a substrate was determined using a β-Glucanase Assay Kit manufactured by Megazyme. Specifically, 0.1 mL of the culture liquid was added to 0.1 mL of an azo-barley glucan substrate solution, and the obtained solution was enzymatically reacted at 40°C for exactly 10 minutes. Then, 0.6 mL of a stop solution [containing 4% sodium acetate, 0.4% zinc acetate and 80% methyl cellosolve (pH 5)] was added thereto, followed by leaving to stand the obtained solution for 5 minutes to stop the reaction. Subsequently, the obtained solution was centrifuged, and absorbance of the supernatant fluid at 590 nm was determined. One unit of β-glucanase activity was represented as the enzyme amount which produces reducing sugars corresponding to 1 µmol of glucose in 1 minute under the reaction conditions of 40°C and 10 minutes.

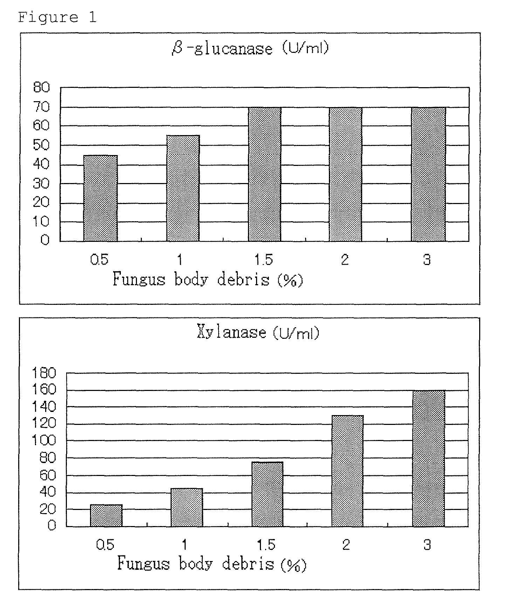

Next, xylanase activity was quantified as an increase in absorbance at 540 nm by reacting reducing sugars produced by enzymatic hydrolysis of xylan derived from "oat spelts" as a substrate with DNS. More specifically, to 1.9 mL of a 1% xylan substrate solution [Xylan, from oat spelts, manufactured by Sigma was dissolved into a 200 mM acetic acid buffer solution (pH 4.5)], 0.1 mL of the culture liquid was added, and the obtained solution was enzymatically reacted at 40°C for exactly 10 minutes.
Then, 4 mL of the DNS reagent (containing 0.75% dinitrosalicylic acid, 1.2% sodium hydroxide, 22.5% potassium sodium tartrate tetrahydrate and 0.3% lactose monohydrate) was added thereto, and the obtained solution was mixed well to stop the reaction. In order to quantify the amount of reducing sugars contained in the reaction stop solution, the reaction stop solution was heated in a boiling-water bath for exactly 15 minutes. Next, the reaction stop solution was cooled to room temperature, and the amount of reducing sugars corresponding to xylose was quantified by determining absorbance at 540 nm. One unit of xylanase activity was represented as the enzyme amount which produces reducing sugars corresponding to 1 µmol of xylose in 1 minute under the reaction conditions of 40°C and 10 minutes. The results are shown in Fig. 1.

### Reference Example 1

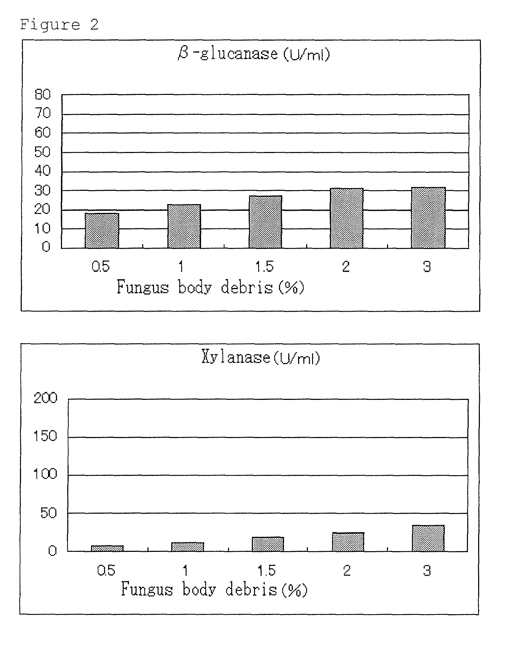

In a Mandel medium, the concentration of crystalline cellulose (trade name Avicel PH101 manufactured by Fluka BioChemika) as a carbon source was adjusted to 1%; and polypeptone as an organic nitrogen source was replaced with fungus body debris obtained in the same manner as in Example 1, and the fungus body debris were added so that their concentrations could be 0.5%, 1.0%, 1.5%, 2.0% and 3.0%, respectively, so as to prepare liquid culture media in the same manner as in Example 1. Trichoderma reesei QM9414 (NBRC 31329) was cultured on a potato dextrose agar medium at 28°C for 7 days to sufficiently form spores. A loopful of the spores was inoculated into each of the liquid culture media, and the fungi were cultured at 28°C and at 180 rpm for 7 days while being shaken. Each of the culture liquids was centrifuged on the seventh day, and the β-glucanase activity and the xylanase activity were determined in the same manner as in Example 1. The results are shown in Fig. 2.

### Example 2

Glycosylation tests of cellulose raw materials were conducted using the culture supernatant fluid obtained in Example 1 (3% copy paper and 1.5% fungus body debris) and the culture supernatant fluid obtained in Reference Example 1 (1% Avicel culture medium and 1.5% fungus body debris). Rice straw and cellulose, "KC Floc" manufactured by NIPPON PAPER Chemicals, CO., LTD. were prepared as the cellulose raw materials to be used for glycosylation. The rice straw was delignified by the following method.

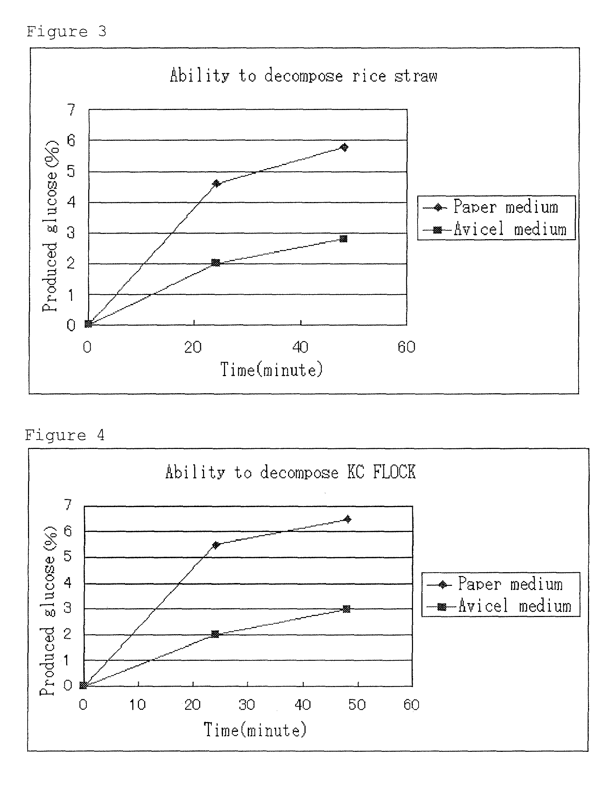

The rice straw was pulverized and was then suspended in 0.3 N NaOH. The suspension was treated at 120°C for 15 minutes and was adequately washed with water and dried. The glycosylation of the cellulose material was measured as follows: a solution (i.e., a solution of 8% cellulose raw material) which was composed of 0.8 g of the cellulose raw material, 9.0 mL of the culture supernatant fluid and 0.2 mL of 1 M acetic acid buffer (pH 4.8) was shaken at 50°C and at pH 4.8 for 48 hours to be glycosylated, and the produced glucose was determined with Glucose CII-Test Wako (Wako Pure Chemical Industries, Ltd.). The results are shown in Figs. 3 and 4.

### Example 3

The residue of beer was collected in the course of manufacturing of beer and was then treated in an aqueous solution of 0.3N sodium hydroxide at 121°C for 15 minutes in an autoclave so as to remove lignin therefrom, and was then sufficiently washed with water and dried.

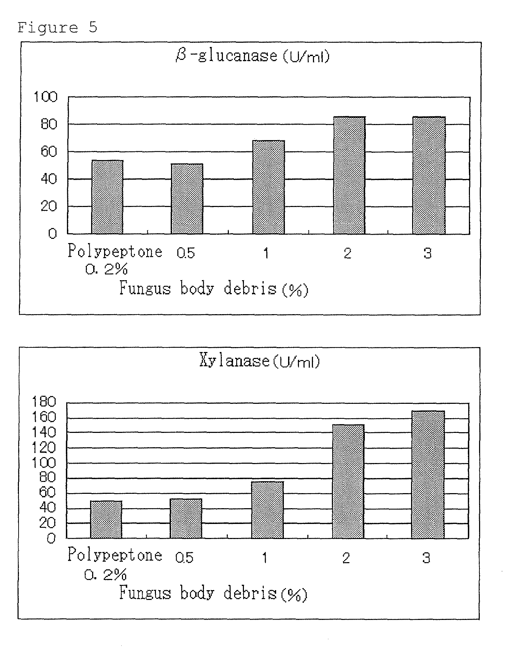

Trichodermal reesei QM9414 (NBRC 31329) was cultured on a potato dextrose agar medium at 28°C for 7 days to form sufficient spores. In a Mandel medium, crystalline cellulose as a carbon source was replaced with 3% of the above-delignified residue of beer (3 g/100 mL); 1% of ammonium sulfate as an inorganic nitrogen source was added; and the organic nitrogen source was replaced with 0.2% of polypeptone, or the fungus body debris obtained in the same manner as in Example 1, and the fungus body debris was added so that their concentrations could be 0.5%, 1.0%, 2.0% and 3.0%, respectively. The resultant liquid culture media were adjusted with phosphoric acid or sodium hydroxide to adjust their pH to 4.8. Then, 100 mL of each of the liquid culture media was poured into a 500 mL baffled Erlenmeyer flask. Then, a loopful of the cultured Trichoderma reesei was inoculated into the liquid culture medium and was cultured at 28°C and at 180 rpm for 7 days. The culture liquid was centrifuged on the seventh day, and the β-glucanase activity and the xylanase activity of the supernatant fluid were determined in the same manner as in Example 1. The results are shown in Fig. 5.

### Example 4

Mugi tea grains (manufactured by Asahi Beer Malt Ltd.) and boiled water were used to make Mugi tea. The Mugi tea as an aqueous solution was removed, the resulting residue was washed with water and dried to obtain the residue of Mugi tea extract.

The resulting residue of Mugi tea extract was pulverized and was then treated in an aqueous solution of 0.3N sodium hydroxide at 121°C for 15 minutes in an autoclave so as to remove lignin therefrom, and was then sufficiently washed with water and dried.

The resulting residue of Mugi tea extract was treated in an aqueous solution of 0.3N sodium hydroxide at 121°C for 15 minutes in an autoclave so as to remove lignin therefrom, and was then sufficiently washed with water and dried.

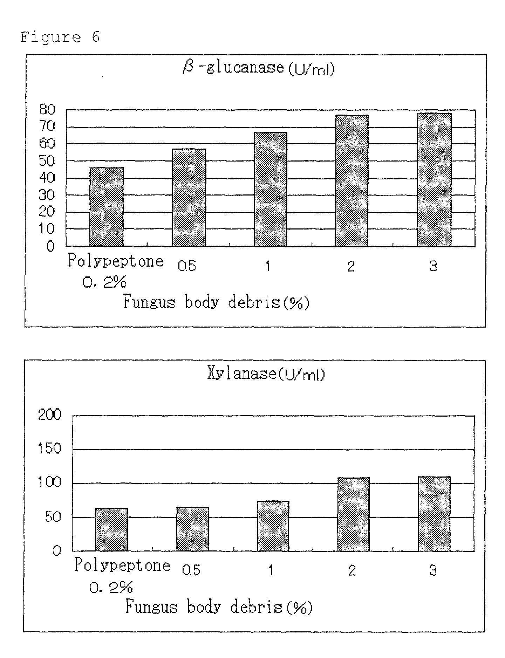

Trichodermal reesei QM9414 (NBRC 31329) was cultured on a potato dextrose agar medium at 28°C for 7 days to form sufficient spores. A culture solution was obtained in the same manner as in Example 3, except that crystalline cellulose as a carbon source in a Mandel medium was replaced with 5% of the above-delignified residue of Mugi tea extract (3 g/100 mL). The enzymatic activity of the obtained culture liquid was determined. The results are shown in Fig. 6.

### Example 5

Wheat bran (Showa Sangyo Co., Ltd.) was pulverized and was then treated in an aqueous solution of 0.3N sodium hydroxide at 121°C for 15 minutes in an autoclave so as to remove lignin therefrom, and was then sufficiently washed with water and dried.

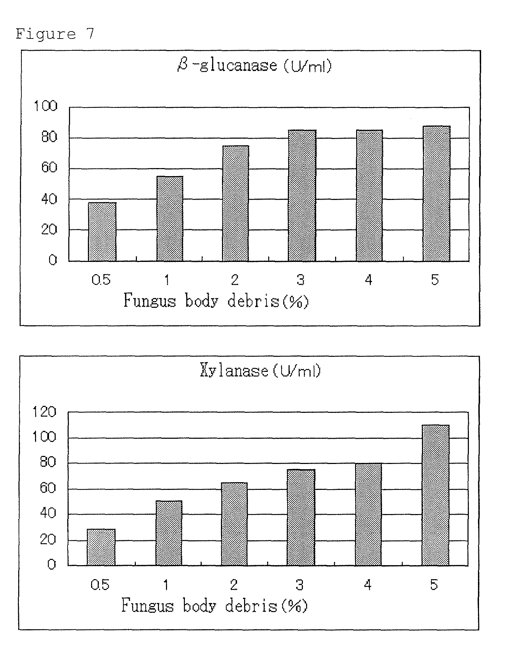

Trichodermal reesei QM9414 (NBRC 31329) was cultured on a potato dextrose agar medium at 28°C for 7 days to form sufficient spores. In a Mandel medium, crystalline cellulose as a carbon source was replaced with 5% of the above-delignified wheat bran (5 g/100 mL); 1% of ammonium sulfate as an inorganic nitrogen source was added; and polypeptone as an organic nitrogen source was replaced with the fungus body debris obtained in the same manner as in Example 1, and the fungus body debris was added so that their concentrations could be 0.5%, 1.0%, 2.0%, 3.0%, 4.0% and 5.0%, respectively. The resultant liquid culture media were adjusted with phosphoric acid or sodium hydroxide to adjust their pH to 4.8. Then, 100 mL of each of the liquid culture media was poured into a 500 mL baffled Erlenmeyer flask. Then, a loopful of the cultured Trichoderma reesei was inoculated into the liquid culture medium and was cultured at 28° and at 180 rpm for 7 days. The culture liquid was centrifuged on the seventh day, and the β-glucanase activity and the xylanase activity of the supernatant fluid were determined in the same manner as in Example 1. The results are shown in Fig. 7.

### Example 6

A crushing machine ("Hammer Mill" manufactured by Amos) was used to crush apples ("Fuji"), and then, an apple juice-extracting machine ("Press Roll Filter" manufactured by Tsukishima-Andritz) was used to extract the juice of the crushed apples. The marc of apple was collected from the juice-extracting machine and was washed with water and dried.

The resulting marc of apple was treated in an aqueous solution of 0.3N sodium hydroxide at 121°C for 15 minutes in an autoclave so as to remove lignin therefrom, and was then sufficiently washed with water and dried. The dried marc of apple was ground into portions with uniform sizes for use.

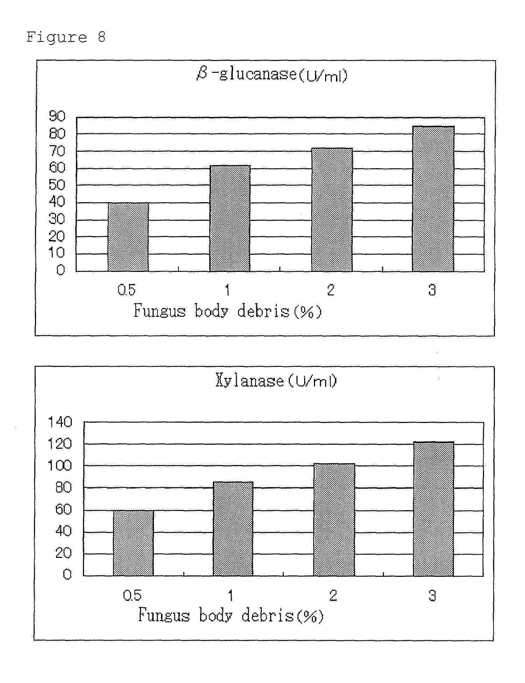

Trichodermal reesei QM9414 (NBRC 31329) was cultured on a potato dextrose agar medium at 28°C for 7 days to form sufficient spores. In a Mandel medium, crystalline cellulose as a carbon source was replaced with 4% of the above-delignified marc of apple (4 g/100 mL); 1% of ammonium sulfate as an inorganic nitrogen source was added; and polypeptone as an organic nitrogen source was replaced with the fungus body debris obtained in the same manner as in Example 1, and the fungus body debris was added so that their concentrations could be 0.5%, 1.0%, 2.0% and 3.0%, respectively. The resultant liquid culture media were adjusted with phosphoric acid or sodium hydroxide to adjust their pH to 4.8. Then, 100 mL of each of the liquid culture media was poured into a 500 mL baffled Erlenmeyer flask. Then, a loopful of the cultured Trichoderma reesei was inoculated into the liquid culture medium and was cultured at 28° and at 180 rpm for 7 days. The culture liquid was centrifuged on the seventh day, and the β-glucanase activity and the xylanase activity of the supernatant fluid were determined in the same manner as in Example 1. The results are shown in Fig. 8.

### Reference Example 2

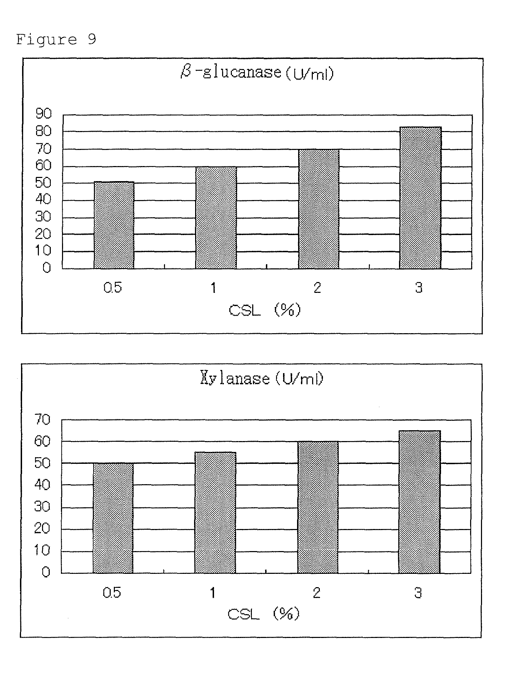

Trichodermal reesei QM9414 (NBRC 31329) was cultured on a potato dextrose agar medium at 28°C for 7 days to form sufficient spores. In a Mandel medium, crystalline cellulose as a carbon source was replaced with 3% of copy paper (3 g/100 mL); 1% of ammonium sulfate as an inorganic nitrogen source was added; and polypeptone as an organic nitrogen source was replaced with corn steep liquor (CSL), and the CSL was added so that their concentrations could be 0.5%, 1.0%, 2.0% and 3.0%, respectively. The resultant liquid culture media were adjusted with phosphoric acid or sodium hydroxide to adjust their pH to 4.8. Then, 100 mL of each of the liquid culture media was poured into a 500 mL baffled Erlenmeyer flask. Then, a loopful of the cultured Trichoderma reesei was inoculated into the liquid culture medium and was cultured at 28° and at 180 rpm for 7 days. The culture liquid was centrifuged on the seventh day, and the β-glucanase activity and the xylanase activity of the supernatant fluid were determined in the same manner as in Example 1. The results are shown in Fig. 9.

### Reference Example 3

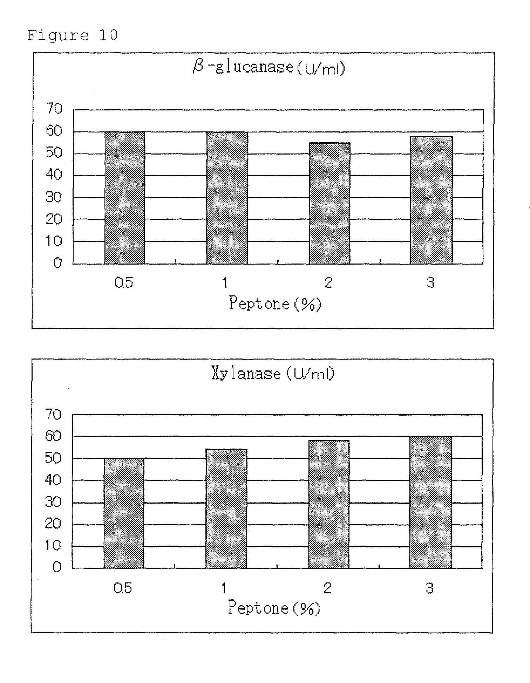

Trichodermal reesei QM9414 (NBRC 31329) was cultured on a potato dextrose agar medium at 28°C for 7 days to form sufficient spores. In a Mandel medium, crystalline cellulose as a carbon source was replaced with 3% of copy paper (3 g/100 mL); 1% of ammonium sulfate as an inorganic nitrogen source was added; and polypeptone as an organic nitrogen source was added so that the polypeptone concentrations could be 0.5%, 1.0%, 2.0% and 3.0%, respectively. The resultant liquid culture media were adjusted with phosphoric acid or sodium hydroxide to adjust their pH to 4.8. Then, 100 mL of each of the liquid culture media was poured into a 500 mL baffled Erlenmeyer flask. Then, a loopful of the cultured Trichoderma reesei was inoculated into the liquid culture medium and was cultured at 28° and at 180 rpm for 7 days. The culture liquid was centrifuged on the seventh day, and the β-glucanase activity and the xylanase activity of the supernatant fluid were determined in the same manner as in Example 1. The results are shown in Fig. 10.

### INDUSTRIAL APPLICABILITY

β-Glucanase and xylanase extremely useful for glycosylation of natural cellulosic resources such as rice straws and the like. can be simultaneously and highly produced, so as to be used for production of biomass ethanol in which ethanol is produced from the cellulosic resources.

## Claims

1. A method for producing β-glucanase and xylanase, comprising the step of culturing a microorganism classified under the genus Trichoderma by using a liquid culture medium which contains fungus body debris as an organic nitrogen source.

2. The method for producing β-glucanase and xylanase, according to Claim 1, wherein the concentration of the fungus body debris in the liquid culture medium is not less than 1% W/V.

3. The method for producing β-glucanase and xylanase, according to Claim 1 or 2, wherein the concentration of the fungus body debris in the liquid culture medium is from 2 to 10% W/V.

4. The method for producing β-glucanase and xylanase, according to any of Claims 1 to 3, wherein a raw material for the fungus body debris is a microorganism classified under the genus Trichoderma.

5. The method for producing β-glucanase and xylanase, according to any of Claims 1 to 4, wherein the microorganism classified under the genus Trichoderma is Trichoderma reesei.

6. The method for producing β-glucanase and xylanase, according to any of Claims 1 to 5, wherein the liquid culture medium further contains a natural cellulose material as a carbon source, and ammonia nitrogen or amino nitrogen as a nitrogen source.

7. The method for producing β-glucanase and xylanase, according to any of Claims 1 to 6, wherein the concentration of the natural cellulose material in the liquid culture medium is not less than 2% W/V.

8. The method for producing β-glucanase and xylanase, according to Claim 6 or 7, wherein the natural cellulose material is at least one kind selected from the group consisting of pulp, residue of beer, residue of Mugi tea extract, wheat bran and a marc of apple.

9. The method for producing β-glucanase and xylanase, according to any of Claims 1 to 8, wherein the fungus body debris is added to the liquid culture medium in the course of culture.

10. A liquid, culture medium containing fungus body debris as an organic carbon source, for use in culture of a microorganism classified under the genus Trichoderma.

11. The liquid culture medium according to Claim 10, containing not less than 1% W/V of the fungus body debris.

12. β-Glucanase and xylanase, produced by the method according to any one of Claims 1 to 9.

13. A method for decomposing or glycosylating a cellulosic resource, **characterized by** using the β-glucanase and the xylanase according to Claim 12.
